# EUROPEAN PATENT APPLICATION

(11) **EP 3 674 702 A1**
(43) Date of publication of application: **01.07.2020**
(21) Application number: 19216844.1
(22) Date of filing: 17.12.2019
(51) Int. Cl.: G01N 27/414, C12Q 1/00, G01N 33/543

(54) **METHOD FOR SEQUENCING A POLYNUCLEOTIDE USING A BIOFET**

(30) Priority: 27.12.2018 EP 18248045
(71) Applicant: Imec VZW, 3001 Leuven (BE)
(72) Inventor: MARTENS, Koen, 3001 Leuven (BE); VAN DORPE, Pol, 3001 Leuven (BE); JANS, Karolien, 3001 Leuven (BE); WORONOFF, Gabrielle, 3001 Leuven (BE)
(74) Representative: DenK iP

(57) **Abstract**

A method for sequencing a template polynucleotide, comprising the steps of:
a) providing a sensor (1) comprising:
- an active region comprising a source region, a drain region, and a channel region (2),
- a dielectric region (3) on the channel region (2),
- a polymerase (4) coupled to the dielectric region (3), the polymerase having an active site (5), the polymerase (4) being separated from the dielectric region (3) by a gap,
- one or more sensitizing means,
- a fluidic gate region (8) to which the polymerase (4) is exposed,
- a template polynucleotide bound to a primer, the template polynucleotide being bound to the polymerase (4),
b) exposing the polymerase (4) to one or more nucleotide polyphosphates; and
c) electrically monitoring changes in the channel electrical properties.

## Description

### Technical field of the disclosure

The present disclosure relates to the field of polynucleotide sequencing.

### Background of the disclosure

Biosensors based on the detection of a single molecule by an enzyme have been described. These devices are important as they can potentially be adapted to allow massively parallel bio-sensing. Applications where such devices are of interest are for instance polynucleotide sequencing, such as DNA sequencing.

WO2014150392 discloses methods for nucleic acid sequencing operating by duplicating a DNA template by using a DNA polymerase, and by monitoring the incorporation of nucleotides in a newly forming DNA strand with electronic sensing elements. These methods aim at increasing the signal generated in individual nucleotide incorporation during DNA sequencing. In general, the methods involve detecting a signal that is associated with the cleavage of polyphosphate chains released from nucleoside polyphosphates incorporated during template-directed primer extension reaction. The nucleoside polyphosphate used include a chain of three or more phosphates and a terminal group. The incorporation reaction is carried out by the polymerase in the presence of a phosphatase enzyme. As a result, in addition to the cleavage of an alpha-beta phosphate bond by the polymerase, which would also happen in absence of the phosphatase enzyme, the cleavage of at least one additional phosphate bond of the polyphosphate chain is obtained. By cleaving at least two phosphate bonds per incorporation, these methods aim at increasing the sensitivity of the detection (typically a pH change detection) by generating two protons where only one proton would be generated if no phosphatase enzyme was used and only one phosphate bond was cleaved. The terminal group is necessary due to the presence of the phosphatase during the sequencing. Without that terminal group, the phosphatase would cleave the polyphosphate chain in solution, before the nucleotide bearing it would have had the chance to interact with the DNA polymerase.

There is, therefore, a need in the art for alternative methods, not necessitating the labor-intensive synthesis of nucleoside polyphosphate comprising a terminal group, and not necessitating the use of phosphatase.

### Summary of the disclosure

It is an object of the present disclosure to provide good methods for sequencing a template polynucleotide.

The methods according to embodiments of the present disclosure are particularly advantageous for FET-based single molecule sensing (e.g. for the sequencing of a single target polynucleotide)

It is an advantage of embodiments of the present disclosure that the methods can have a good sensitivity. This is obtained by reducing the screening effect operated by the electrolyte on the nucleotide polyphosphates being incorporated during the sequencing. In embodiments, this can be done while keeping the screening effect operated by the electrolyte on the non-target molecules.

It is another advantage of embodiments of the present disclosure that the labor-intensive synthesis of nucleoside polyphosphate comprising a terminal group is not necessary.

It is an advantage of embodiments of the present disclosure that the use of a phosphatase is not necessary.

It is another advantage of embodiments of the present disclosure that the sensors used can be mass-produced.

It is yet another advantage of embodiments of the present disclosure that the sensors used can be produced at low cost.

It is yet a further advantage of embodiments of the present disclosure that a high integration density of the sensor used can be achieved.

In a first aspect, the present disclosure relates to a method for sequencing a template polynucleotide, comprising the steps of:
a) Providing a sensor comprising:
   - An active region comprising a source region, a drain region, and a channel region between the source region and the drain region,
   - A dielectric region on the channel region,
   - A polymerase coupled to the dielectric region, the polymerase having an active site for template polynucleotide entry, nucleotide polyphosphate entry, and newly synthesized polynucleotide output, the polymerase being separated from the dielectric region by a gap,
   - One or more sensitizing means,
   - The fluidic gate region to which the polymerase is exposed,
   - A template polynucleotide bound to a primer, the template polynucleotide being bound to the polymerase,
b) Exposing the polymerase to one or more nucleotide polyphosphates; and
c) Electrically monitoring changes in a channel electrical properties,
   wherein the one or more sensitizing means are selected from;
   o an aqueous electrolyte filling said gap and at least part of a fluidic gate region, the aqueous electrolyte having a conductivity below 1 S/m, preferably below 0.3 S/m,
   ∘ an electrolyte-screening layer bound to the dielectric region,
   ∘ a maximal distance of 3 nm or less, preferably 2 nm or less between the part of the polymerase closest to the dielectric region and the dielectric region,

Step c operates while step b is still being performed.

The present inventors realized that methods for sequencing a template polynucleotide making use of biosensors, and especially FET (bioFET) silicon-based biosensors, suffer from a low sensitivity due to electrolyte screening. Indeed, they realized that the presence of an electrolyte in the gap between the polymerase and the dielectric region of the sensor severely weakens the electric field generated by the target molecule. The present inventors disclose herein three analogous solutions to this problem, each solution operating by reducing the influence of an electrolyte present between the nucleotide polyphosphate and the channel of the sensor during the sensing event. Any of these three solutions or a combination thereof permit the use of a simplified sequencing method while keeping a good sensitivity. A first solution to this problem is to use an aqueous electrolyte filling said gap and at least part of the fluidic gate region, the aqueous electrolyte having a conductivity below 0.3 S/m. Method for sequencing a template polynucleotide traditionally operates in an electrolyte. This electrolyte can, for instance, be a biological sample or a buffered salt solution. By simply using an electrolyte of lower salinity, e.g. by diluting the electrolyte traditionally used, the sensitivity of the sensor toward the nucleotide polyphosphate can in some embodiments be sufficiently increased to enable good sensitivity of the method according to the present disclosure. However, for convenience, one would sometimes like to operate the sensor at the relatively high physiological salinity level of typical biological samples. Working at the salinity level of typical biological samples also has the advantage of reducing the electrical impact of non-specific binding to the bioFET surface. Due to increased screening, non-specifically bound charges result in a weaker signal. For this reason, two alternative sensitizing means are presented herein which can be used together or not, and in combination or not with the use of an aqueous electrolyte of low conductivity. The first alternative makes use of a sensor and a biosensing device having an electrolyte screening layer reducing the amount of electrolyte present between the channel of the sensor and the nucleotide polyphosphate at the time of its incorporation in the growing new polynucleotide strand. The second alternative makes use of a short distance between the polymerase and the dielectric region, thereby reducing the amount of electrolyte present between the channel of the sensor and the nucleotide polyphosphate at the time of its incorporation.

Typically, each phosphate group of each nucleotide polyphosphate is charged negatively. During steps b) and c), i.e. during the sequencing, these charges are inside the polymerase (the nucleotide polyphosphate is enveloped by a binding pocket of the polymerase active site), thereby reducing the screening effect (by repelling the electrolyte) that would be present if the detection of the nucleotide polyphosphate was occurring outside of the polymerase.

In embodiments, the electrolyte-screening layer covers part of the polymerase while leaving the active site uncovered.

In embodiments, the height of the electrolyte-screening layer is such that more than half of the height of the polymerase is covered.

In embodiments, the electrolyte-screening layer may comprise a lipid bilayer.

In embodiments, the lipid bilayer may be a cell membrane.

In embodiments, the height (h) of the electrolyte-screening layer may be such that from 2 to 10 nm of the height (H) of the polymerase is covered.

In embodiments, the maximal distance between the part of the polymerase closest to the dielectric region and the dielectric region is 3 nm or less, 2 nm or less, or 1 nm or less.

In embodiments, the one or more nucleotide polyphosphates do not comprise a terminal blocking group.

In embodiments, the one or more nucleotide polyphosphates have the general formula: 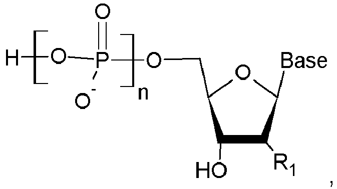 or an ionic form thereof,
wherein n is at least 3, R₁ is either H or OH and the base is selected from any of the natural or non-natural nucleobases or nucleobase analogs. Such natural or non-natural nucleobases or nucleobase analogs are described below. Preferably, the base is selected from adenine, guanine, cytosine, thymine, and uracil.

In embodiments, the one or more nucleotide polyphosphates may comprise at least a nucleotide polyphosphate comprising a polyphosphate chain of four or more phosphates.

In embodiments, step b) may comprise exposing the polymerase to a single type of nucleotide polyphosphate of a first base, and the method repeats (or in other words, cycles) steps b) and c), by substituting the single type of nucleotide polyphosphate of a first base by a single type of nucleotide polyphosphate of a different base than the base in the just used nucleotide polyphosphate for each new repeat (or cycle).

In embodiments, step b) may comprise simultaneously exposing the polymerase to a first type of nucleotide polyphosphate of a first base, and to a second type of nucleotide polyphosphate of a second base, different from the first base, the first type of nucleotide polyphosphate comprising a polyphosphate chain of at least one more phosphates, preferably at least two more phosphates than a polyphosphate chain of the second type of nucleotide polyphosphate, and wherein the method repeats (or in other words, cycles) steps b) and step c), by substituting the nucleotide polyphosphates just used by nucleotide polyphosphates of other bases, said bases being different from each other, for each new repeat (or cycle) of steps b and c.

In embodiments, step b) may comprise simultaneously exposing the polymerase to four types of nucleotide polyphosphate, each type having a different base and a different number of phosphates in a polyphosphate chain thereof.

The above objective is accomplished by a method according to embodiments of the present disclosure.

Particular and preferred aspects of the disclosure are set out in the accompanying independent and dependent claims. Features from the dependent claims may be combined with features of the independent claims and with features of other dependent claims as appropriate and not merely as explicitly set out in the claims.

Although there has been constant improvement, change and evolution of methods in this field, the present concepts are believed to represent substantial new and novel improvements, including departures from prior practices, resulting in the provision of more efficient, stable and reliable devices of this nature.

The above and other characteristics, features and advantages of the present disclosure will become apparent from the following detailed description, taken in conjunction with the accompanying drawings, which illustrate, by way of example, the principles of the disclosure. This description is given for the sake of example only, without limiting the scope of the disclosure. The reference figures quoted below refer to the attached drawings.

### Brief description of the drawings

Figs. 1 to 4 are schematic illustrations of vertical cross-sections of embodiments of sensors that can be used in embodiments of the present disclosure.
Fig. 5 is a schematic illustration of vertical cross-sections of sensors, illustrating an aspect of the present disclosure.
Fig. 6 shows a graph of the voltage shift (mV) observed when polymerase captures a charged substrate during A) simulations of embodiments of the present invention in which the dimension correspond to the varied distance between enzyme and dielectric (see fig 8A) and B) simulations of embodiments of the present disclosure in which the dimension correspond to the varied electrolyte screening layer thickness (see fig 8B).
Fig. 7 shows a 3D illustration of a simulated sensor usable according to an embodiment of the present disclosure.
Fig. 8 is a schematic illustration of parameters used to simulate a sensor usable in embodiments of the present disclosure.
Fig.9 shows examples of nucleotide polyphosphates usable in embodiments of the present disclosure.

In the different figures, the same reference signs refer to the same or analogous elements.

### Description of illustrative embodiments

The present disclosure will be described with respect to particular embodiments and with reference to certain drawings but the disclosure is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the disclosure.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the disclosure described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present disclosure, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present disclosure. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the disclosure, various features of the disclosure are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed disclosure requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this disclosure.

Furthermore, while some embodiments described herein include some but not other features included in other embodiments, combinations of features of different embodiments are meant to be within the scope of the disclosure, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the disclosure.

In the description provided herein, numerous specific details are set forth. However, it is understood that embodiments of the disclosure may be practiced without these specific details. In other instances, well-known methods, structures and techniques have not been shown in detail in order not to obscure an understanding of this description.

The disclosure will now be described by a detailed description of several embodiments of the disclosure. It is clear that other embodiments of the disclosure can be configured according to the knowledge of persons skilled in the art without departing from the technical teaching of the disclosure, the disclosure being limited only by the terms of the appended claims.

The first aspect of the present disclosure relates to a method for sequencing a template polynucleotide.

The template polynucleotide comprises at least two nucleotides covalently attached together. A template polynucleotide of the present disclosure will typically contain phosphodiester bonds, although in some cases, template polynucleotides may have alternate backbones, comprising, for instance, phosphorothioate, phosphoramide, phosphorodithioate, and peptide nucleic acid linkages and backbones. Other suitable polynucleotides include those with positive backbones, or non-ribose backbones, including those described in U.S. Pat. Nos. 5,034,506 and 5,235,033. The template polynucleotide may also be modified in other ways, such as by the attachment of labels, such as dyes, the inclusion of heteroatoms, or the substitution with functional groups which will still allow for recognition by the polymerase and for base pairing. Preferably, no labels are present on the template polynucleotide.

The present disclosure provides methods for identifying the sequences of template polynucleotides. Typically, the template polynucleotide is the compound for which a complementary sequence is formed during the polymerase reaction. In embodiments, the template polynucleotide may be circular or linear. The template polynucleotide can be RNA, DNA, or can be a non-natural DNA analog or RNA analog. Any template polynucleotide suitable for replication by a polymerase can be used.

In embodiments, the template polynucleotide may be a linear double or single-stranded nucleic acid sequence. In other embodiments, the template polynucleotide may be a circular or functionally circular construct permitting redundant processing of the same nucleic acid sequence by the synthesis complex. Use of such circular constructs has been described in, e.g., U.S. Pat. No. 7,901,889, and U.S. Patent No. 7,315,019. Other functional circular constructs are also described in US Pat. App. Pub. No. 20090298075 the full disclosures of each of which are incorporated herein by reference in their entirety for all purposes and in particular for all teachings related to template polynucleotide constructs. Such alternate constructs include template polynucleotides that possess a central double-stranded part that is linked at each extremity by a linking oligonucleotide, such as a hairpin loop segment. Such molecular structures not only provide the ability to repeatedly replicate a single molecule (and thus sequence that molecule), but also provide for additional redundancy by replicating both the antisense and the sense parts of the double-stranded part. In sequencing applications, such redundant sequencing is advantageous as they improve sequence accuracy.

In embodiments, genomic DNA is obtained from a sample and fragmented for use in methods of the disclosure. The fragments may be double or single-stranded and may further be modified in accordance with any methods described herein or known in the art. Template polynucleotides may be formed by fragmenting source polynucleotides, such as genomic DNA, using any method known in the art. In embodiments, shear forces during lysis and extraction of genomic DNA generate such fragments. Methods of fragmentation utilizing restriction endonucleases are also encompassed by the present disclosure.

The sample from which the DNA may be obtained can, for instance, be bodily fluids (e.g. urine, blood, serum, saliva, lymph, vaginal or anal secretions, semen or perspiration) or cells of any organism. Mammalian samples are preferred. Human samples are particularly preferred. Other sample examples include environmental samples (e.g. agricultural, air, soil or water samples); research samples (the sample may, for instance, be the products of the amplification of a polynucleotide, including both signal and target amplification, such as PCR amplification reactions; purified samples, such as purified genomic DNA, RNA preparations, raw samples (e.g. genomic virus, DNA, bacteria)); biological warfare agent samples; as will be appreciated by those in the art, any experimental manipulation may have been done on the samples.

Target polynucleotides may be generated from a source polynucleotide, such as genomic DNA, by fragmentation to form fragments of a certain size. The target polynucleotide can, for instance, have a length of from 10 to 50000 nucleotides, or from 10 to about 20000 nucleotides. In embodiments, the fragments may have a length of from 50 to 2000 nucleotides.

The first step of the method (step a) is to provide a sensor.

The sensor may be for sensing a nucleotide polyphosphate. Typically, the sensor is for sensing a single nucleotide polyphosphate at a time. The nucleotide polyphosphate is charged. The presence of this charge close to the channel is felt by the sensor. The nucleotide polyphosphates are in a liquid medium, typically an electrolyte. In embodiments, the liquid medium may be an aqueous electrolyte medium such as an aqueous buffered salt solution. In an embodiment of the present disclosure, the aqueous electrolyte has a conductivity below 0.3 S/m. In some embodiments, the device of the present disclosure is particularly well suited to detect the nucleotide polyphosphate (at the time of its incorporation in the new growing polynucleotide strand) in a biological fluid. In the prior art, the detection of a target molecule in a biological fluid with a BioFET (Field Effect Transistor-based biosensor) was problematic due to the high salinity, and therefore the high screening effect of typical biological samples. A problem that embodiments of the present disclosure addresses.

The sensor of the present disclosure is typically referred to as a BioFET. BioFETs sense molecules as charges or dielectric displacements close to their gate dielectric. These transistors do not have their solid gate electrode directly in contact with the gate dielectric. Instead, they have a liquid electrolyte gate in contact with the gate dielectric and the solid gate electrode is in electrical contact with the liquid electrolyte gate. The target polynucleotide is located in this liquid electrolyte gate and are deteted near the gate dielectric surface. The molecular charge or dielectric displacement near the gate dielectric causes a change in the current running through the semiconductor channel of the device as dictated by the disturbance in the electrical potential landscape these molecules cause.

The sensor comprises:
- An active region comprising a source region, a drain region, and a channel region between the source region and the drain region,
- A dielectric region on the channel region,
- A polymerase coupled to the dielectric region, the polymerase having an active site for template polynucleotide entry, nucleotide polyphosphate entry, and newly synthesized polynucleotide output, the polymerase being separated from the dielectric region by a gap,
- One or more sensitizing means selected from;
   ∘ an aqueous electrolyte filling said gap and at least part of the fluidic gate region, the aqueous electrolyte having a conductivity below 0.3 S/m,
   ∘ an electrolyte-screening layer bound to the dielectric region,
   ∘ a maximal distance of 3 nm or less, preferably 2 nm or less, more preferably 1 nm or less, between the part of the polymerase closest to the dielectric region and the dielectric region,
- A fluidic gate region to which the polymerase is exposed, and
- A template polynucleotide bound to a primer, the template polynucleotide being bound to the polymerase,

These elements can be seen as forming a field effect transistor (FET).

The active region typically comprises a semiconductor material such as, but not limited to, silicon.

The active region comprises a channel region. In embodiments, the channel region may have a length of 10 to 150 nm. In embodiments, the channel region may have a width of 3 to 20 nm. In embodiments, the channel region may have a height of 5 to 40 nm.

The source region and the drain region are typically regions of higher conductivity of the active region. The source region and the drain region are separated by the channel region, which has a lower conductivity than the source region and the drain region. The source region and the drain region can for instance be formed by doping.

The dielectric region typically comprises a dielectric material, such as for instance a silicon oxide (e.g. SiO₂) or a high-k dielectric (e.g. HfO₂).

Any suitable polymerase may be used in the methods of the present disclosure. Examples of suitable polymerases are RNA-dependent RNA polymerases, RNA-dependent DNA polymerases, DNA-dependent DNA polymerases, and DNA-dependent RNA polymerases, (i.e. reverse transcriptases).

DNA polymerases can be classified based upon their phylogenetic relationships. Examples are E. coli Pol I (class A), E. coli Pol II (class B), E. coli Pol III (class C), Euryarchaeotic Pol II (class D), human Pol beta (class X), and eukaryotic RAD30/xeroderma pigmentosum variant and E. coli UmuC/DinB (class Y). Burgers et al. (2001) "Eukaryotic DNA polymerases: proposal for a revised nomenclature" J Biol Chem. 276(47):43487-90 offers a review of recent nomenclature. The general action mechanisms for many polymerases have been published. The sequences of hundreds of polymerases have also been published, and the crystal structures for many of these polymerases have been determined or can be inferred by comparison with the crystal structures of homologous polymerases. For example, the crystal structure of Φ29, a type of polymerase suitable for use in the present disclosure, is available. Φ29 is advantageous as it is known to be able to process nucleotide polyphosphate having more than 3 phosphate groups.

In addition to polymerases occurring naturally, chimeric polymerases made from a plurality of different sources can be used. For example, Φ29 polymerases made by taking sequences from more than one parental polymerase into account can be used to produce by mutation further polymerases usable in the present disclosure.

Other usable DNA polymerases are those that have been modified to add or suppress a function. For instance, DNA polymerases adapted to eliminate or reduce exonuclease activities (such activities interfere with sequencing applications), DNA polymerases adapted to alter branch fraction and translocation (see US2010075332), to increase photostability (see US2016017413), and to improve surface-immobilized enzyme activities (see WO 2007/075987). These known polymerases can be adapted as is well known in the art to improve the stability of the closed polymerase-DNA complex, to decrease branching fraction formation, and/or to alter reaction rate constants. In embodiments, the polymerase may be modified in order to more effectively incorporate the nucleotide polyphosphates of the present disclosure, e.g. those having four or more phosphates in their polyphosphate chain. Enzymes mutated to more readily accept nucleotide analogs having such properties are known, for example from US20120034602, from US 20100093555, from US 20110189659, from US20100112645, from US 2008/0108082, and from US20110059505 which sections relating to enzymes mutated to more readily accept nucleotide analogs are incorporated herein by reference in their entirety.

Polymerases usable in the present disclosure, as such or after modifications, are commercially available. For example, DNA polymerase I is available from Sigma Aldrich. Human DNA Polymerase Beta can be purchased from R&D systems. The Klenow fragment of DNA Polymerase I is available in both protease digested and recombinant versions, from Sigma Aldrich. The Φ29 DNA polymerase is available from e.g., Epicentre. Reverse transcriptase, Poly A polymerase, Sequenase, T4 DNA polymerase, T7 DNA polymerase, SP6 DNA polymerase, and a variety of thermostable DNA polymerases (hot start, Taq, titanium Taq, etc.) are also commercially available. Further examples of commercial DNA polymerases are GoTaq® Flexi DNA Polymerase, available from Promega; Phusion™ High-Fidelity DNA Polymerase, available from New England Biolabs; RepliPHI™ Φ29 DNA Polymerase, available from Epicentre Biotechnologies; KOD HiFi DNA Polymerase, available from Novagen; and PfuUltra™ Hotstart DNA Polymerase, available from Stratagene.

DNA polymerases that are preferred substrates for mutation to increase closed complex stability, decrease branching fraction, or alter reaction rate constants include exonuclease deficient Taq polymerases, Taq polymerases, E. coli DNA Polymerase 1, reverse transcriptases, Klenow fragment, Φ 29-related polymerases including wild type Φ29 polymerase and derivatives thereof such as exonuclease deficient forms, T5 DNA polymerase, T7 DNA polymerase, and RB69 polymerase.

In embodiments, polymerases usable in methods of the present disclosure include a modified recombinant Φ29-type DNA polymerase. For example, the modified recombinant Φ29-type DNA polymerase can be homologous to a wild-type or exonuclease deficient Φ29 DNA polymerase, e.g., as described in U.S. Patent Nos. 5,198,543; 5,001,050; or 5,576,204. In embodiments, the modified recombinant DNA polymerase can be homologous to other Φ29-type DNA polymerases, such as GA-1, B103, Φ15, BS32, M2Y, PZA, Nf, Cp-1 , G1 , PRD1 , SF5, PZE, Cp-5, Cp-7, L17, PR4, PR5, PR722, Φ21, or the likes. For nomenclature, see also, Meijer et al. (2001) "Φ29 Family of Phages" Microbiology and Molecular Biology Reviews, 65(2):261-287. Suitable polymerases are described, for example, in U.S. Patent Applications US8420366B2; and US2010112645.

In other embodiments, the polymerase used in the methods of the present disclosure includes reverse transcriptases or RNA-dependent DNA polymerases. Suitable reverse transcriptases include M-MLV, HIV-1, Telomere Reverse Transcriptase, and AMV. Reverse transcriptases also permit the direct sequencing of RNA substrates such as transfer RNA, messenger RNA, noncoding RNA, micro RNA, ribosomal RNA, or catalytic RNA.

The polymerase has an active site. the term *"active site"* refers to the part of the polymerase involved in the interaction with the substrate(s). It typically comprises a cavity suitable for interacting with the substrate(s). In some case, the active site may comprise several regions such as in the case of a DNA polymerase which comprises a region for ssDNA template (a substrate) entry, a region for nucleotide entry (another substrate), and a region for the output of a newly synthesized dsDNA. Preferably, the polymerase comprises a binding pocket for binding with a substrate.

In embodiments, the active site permits template polynucleotide entry, nucleotide polyphosphate entry, and newly synthesized polynucleotide output.

The polymerase enzymes of the present disclosure generally require a primer, which is usually a short oligonucleotide that is complementary to a portion of the template polynucleotide. The primers of the present disclosure can comprise naturally occurring DNA or RNA oligonucleotides. The primers of the disclosure may also be synthetic analogs. The primers may have alternative backbones as described above for the target polynucleotides of the present disclosure. The primer may also have other modifications, such as the inclusion of heteroatoms, the attachment of labels, such as dyes, or substitution with functional groups which will still allow for base pairing and for recognition by the polymerase. Primers can select tighter binding primer sequences, e.g., GC rich sequences, as well as employ primers that include within their structure non-natural nucleotides or nucleotide analogs, e.g., peptide nucleic acids (PNAs) or locked nucleic acids (LNAs), that can demonstrate higher affinity pairing with the template. The primer can also be selected to influence the kinetics of the polymerase reaction.

For the polymerase to start duplicating the polynucleotide template, typically a cofactor is needed. A typical cofactor is magnesium. Hence, a magnesium salt, such as MgCl₂ can be present in the aqueous electrolyte during the sequencing. The cofactor can be introduced in step a or in step b.

In embodiments, a binding layer may be present on the dielectric region, and the polymerase may be coupled to the dielectric region by being bound to the binding layer.

In embodiments, the binding layer may be a self-assembled monolayer. The self-assembled monolayer can, for instance, be formed of organosilane molecules. Organosilane molecules have the advantage of having a functional group (e.g -SiCl₃ or -SiOR'₃) suitable for attaching covalently to most dielectric material and in particular to oxide materials such as silicon oxide. The organosilanes are chosen so that they possess a functional group permitting attachment to the polymerase (typically via a linker) and/or to the electrolyte-screening layer.

In embodiments, the silane may be of general formula Z-R-X wherein X is a group suitable for attaching to the dielectric region (e.g. X may be SiOR'₃ or R-SiCl₃) wherein R is an organic chain (e.g. having from 1 to 20 carbon atoms) and R' is an alkyl (typically methyl, ethyl or a combination thereof). Z is a functional group suitable for attaching to the polymerase (e.g. via a linker) and/or to the electrolyte-screening layer. Z is advantageously an azido group or an alkyne group (e.g. a strained alkyne group). When Z is an azido group, the polymerase or more typically the linker attached to the polymerase, preferably may have an alkyne group for reacting with the azido group. When Z is an alkyne group, the polymerase or more typically the linker attached to the polymerase, preferably may have an azido group for reacting with the azido group.

An example of silane may be an azido-C₁₋₁₁ alkyl-trialkoxysilane. Examples are for instance azidomethyltriethoxysilane, 2-azidoethyltrimethoxysilane, 2-azidoethyltriethoxysilane, 3-azidopropyltrimethoxysilane, 3-azidopropyltriethoxysilane, 3-azidopropyltris(methoxyethoxy)silane, 3-azidopropylmethyldiethoxysilane, p-azidomethylphenyltrimethoxysilane, 4-azidobutyltri-n-butoxysilane, 5-azidopentyldimethoxyphenylsilane, 6-azidohexyldiethoxybenzylsilane, 7-azidoheptylmethoxydimethylsilane, 8-azidooctylethoxydiethylsilane, and 11-azidoundecyltrimethoxysilane. Such silanes can be either purchased or prepared according to U.S. Pat. No 4,401,598. Shorter silanes, such as azidomethyltrialkoxysilanes, 2-azidoethyltrialkoxysilanes, and 3-azidopropyltrialkoxysilanes are advantageous as they allow a shorter distance (e.g. of 3 nm or below or even 2 nm or below or 1 nm or below) between the polymerase and the dielectric region.

Such azido-C₁₋₁₁ alkyl-trialkoxysilane may be deposited as a self-assembled monolayer by vapor phase deposition at 145°C onto a SiO₂ surface.

In embodiments, the binding layer may be an electrografted layer.

In embodiments, the electrografted layer may be formed from molecules having the general formula Z-R-X' wherein R and Z are as defined above and X' is a group suitable for attaching to the dielectric region by electrografting (e.g. X' may be a diazonium salt).

In embodiments, a linker may be attached to the dielectric region or the binding layer, and the polymerase may be coupled to the dielectric region by being bound to the linker.

The linker is typically of formula A-L-E, wherein A is a functional group capable of attaching to the binding layer (e.g. an alkyne such as a strained alkyne group if the binding layer presents an azido group available for reaction, or an azido group if the binding layer presents an alkyne group (such as a strained alkyne) available for reaction) or to the dielectric region (e.g. an -SiOR'₃ or R-SiCl₃ group), and E is a functional group capable of attaching to the polymerase, eventually after modification of the polymerase.

Prior to attachment of the linker to the polymerase, the polymerase itself might need to be modified. Examples of E are oxiamine, catechol, alkyne (e.g. a strained alkyne), azide, puromycin, and aniline, amongst others.

Below are a few examples of how to attach a linker to an polymerase.

Gilmore et al. (Angew. Chem. Int. Ed. 2006, 45, 5307-5311) describes a method involving the site-specific modification of the N-terminal position of a protein (which could be replaced by the polymerase of the present disclosure), comprising forming a pyruvamide from the terminal amino group of the protein, followed by reaction with a molecule comprising an oxiamine group (where the molecule could be the linker of the present disclosure and the oxiamine could be the functional group E of the linker).

Song et al. (acta biomaterialia 43 (2016) 50-60) describes a method involving the site-specific modification of the N-terminal position of a protein (which could be replaced by the polymerase of the present disclosure), comprising reacting the protein with a molecule comprising a catechol functional group (where the molecule could be the linker of the present disclosure and the catechol could be the functional group E of the linker).

Temming et al. (Org. Biomol. Chem., 2013, 11, 2772) describes a method involving the site-specific modification of the N-terminal position of a protein (which could be replaced by the polymerase of the present disclosure), comprising forming a nitrone group from the terminal amino group of the protein, followed by reaction with a molecule comprising a strained alkyne group (where the molecule could be the linker of the present disclosure and the strained alkyne group could be the functional group E of the linker).

Humenik et al. (ChemBioChem 2007, 8, 1103-1106) describes a method involving the site-specific modification of the C-terminal position of a protein (which could be replaced by the polymerase of the present disclosure), comprising reacting the polymerase with a puromycin linker comprising an azide group (where the puromycin linker could be the linker of the present disclosure, the azide group could be the functional group A of the linker).

EISohly et al. (Acc. Chem. Res. 2015, 48, 1971-1978) describe the use of oxidative coupling strategies for site-selective protein modification. One described method that could be adapted to the present disclosure could involve the attachment of an o-azidophenol group at the N-terminal amino acid of the polymerase, followed by reaction with a linker comprising an aniline group, which can photoreact with the o-azidophenol group, and a silane group, which can react with the dielectric region.

Yet other methods that can be similarly adapted to the present disclosure are disclosed in Chen et al. (Chem. Sci., 2017, 8, 8, 2717), Boutureira et al. (Chem. Rev. 2015, 115, 2174-2195), Rosen et al. (Nature Chemical Biology, 13, July 2017, 697-705), Sasaki et al. (Bioorg. Med. Chem. Lett. 18 (2008) 4550-4553), and Chan et al. (J. Am. Chem. Soc. 2012, 134, 2589-2598).

In order to form a very short linker and thereby enable a maximal distance of 3 nm or less, or even 2 nm or less or 1 nm or less between the part of the polymerase closest to the dielectric region and the dielectric region, on can make use of a DBCO sulfo NHS ester linker of the following formula: 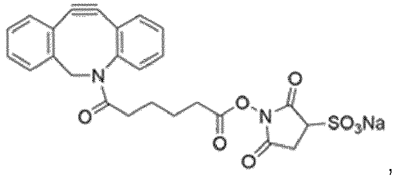 wherein the DBCO group correspond to A in the general formula A-L-E, and wherein the sulfo-NHS corresponds to E in the general formula A-L-E.

With this approach, the length of the linker can be easily adapted by adapting the nature of the chain L linking the DBCO group and the NHS group. An example of a longer linker is a DBCO-PEG4-NHS linker (with a longer 25A spacer) of the following formula:

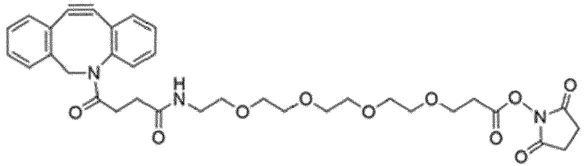

By using a PEG3, a PEG2, or a PEG1 linker L instead, a maximal distance of 3 nm or less, or even 2 nm or less or 1 nm or less, between the part of the polymerase closest to the dielectric region and the dielectric region can be achieved.

The combination of a binding layer selected from azidomethyltrialkoxysilanes, 2-azidoethyltrialkoxysilanes, and 3-azidopropyltrialkoxysilanes with a linker selected from 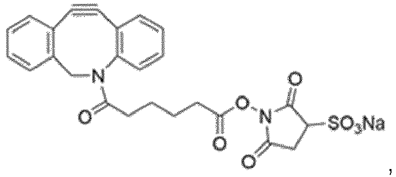 DBCO-PEG1-NHS, DBCO-PEG2-NHS, and DBCO-PEG3-NHS permit to achieve a maximal distance of 3 nm or less, or even 2 nm or less or 1 nm or less, between the part of the polymerase closest to the dielectric region and the dielectric region.

In embodiments, attaching the polymerase to the dielectric region may comprise the steps of i) providing a binding layer on the dielectric region, ii) reacting a linker with the binding layer, iii) and reacting the polymerase with the linker. Between step ii) and step iii), a rinsing step may be performed. For instance, in embodiments, the polymerase can be coupled to the dielectric region by attaching one or multiple amine groups of the polymerase to a surface of the dielectric region covered with an azide SAM making use of amine reactive NHS chemistry combined with click chemistry, typically in a two-step process. The linker is reacted with the azide SAM of the dielectric surface first. The DBCO group of the linker will covalently bind to the azide moiety of the surface. This is followed by a rinsing step to remove all unreacted reagent. Consecutively this is followed by incubating the polymerase in a liquid on the azide covered dielectric surface with linkers attached to azide moieties. In alternative embodiments, attaching the polymerase to the dielectric region may comprise the steps of i) reacting the polymerase with a linker, ii) providing a binding layer on the dielectric region, iii) contacting the dielectric region and the binding layer thereon with the polymerase-linker complex. Preferably, a purification step is performed after step i). For instance, in embodiments, the linker may first be incubated with the polymerase in order for the linker to bind with its NHS group to available amine groups of the polymerase. The mixture may then be purified after which the modified polymerase may be applied to the surface. The modified protein's DBCO group will bind covalently to the azide surface. As another example, A DBCO-L-CHO linker may be reacted to lysine residues of the polymerase via its aldehyde end, thereby forming a Schiff base. Next, the Schiff base may be reduced. The DBCO group may then be reacted (e.g. by click chemistry) with an azide group of the binding layer.

In embodiments, A DBCO-L-CHO linker may be reacted specifically with the N-terminal amine of a polymerase by performing a reductive alkylation at a pH of 6.1 as described in Diao Chen et al., Chem. Sci., 2017, 8, 2717-2722 | 2717. After the attachment of the linker to the N-terminal amine, the modified protein can be reacted (e.g. by click chemistry) with an azide group of the binding layer.

In embodiments, a polymerase functionalized with an azide group may be obtained by recombinant synthesis. For such a purpose, the polymerase may be formed from an expanded genetic code comprising the amber UAG codon. Such a polymerase comprising a non-canonical amino acid can be directly attached to a binding layer comprising an alkyne group (strained or not). Similarly, a polymerase functionalized with an alkyne group (strained or not) may be obtained by recombinant synthesis. For such a purpose, the polymerase may be formed from an expanded genetic code comprising the amber UAG codon. Such a polymerase comprising a non-canonical amino acid can be directly attached to a binding layer comprising an azide group. The resulting polymerase may comprise for instance azido-lysine (AzK) or p-azidophenylalanine (a polymerase functionalized with azido groups). In other embodiments, the resulting polymerase may comprise for instance p-propargyloxyphenylalanine (a polymerase functionalized with an alkyne group).

Other recombinant methods may modify the genetic code of the enzyme in order to bind linkers to specific locations of the enzyme, for instance to cysteines or lysines.

The sensor comprises one or more sensitizing means. These means fulfill the purpose of increasing the sensitivity of the sensor by reducing the screening effect exerted by the electrolyte between the nucleotide polyphosphate being incorporated in the growing new polynucleotide strand and the channel of the sensor. The one or more sensitizing means are selected from
o an aqueous electrolyte filling the gap and at least part of the fluidic gate region, the aqueous electrolyte having a conductivity below 0.3 S/m,
o an electrolyte-screening layer bound to the dielectric region (3), and
o a maximal distance of 3 nm or less, preferably 2 nm or less, yet more preferably 1 nm or less between the part of the polymerase closest to the dielectric region and the dielectric region.

Typically, a gap exists between the dielectric region and the polymerase. This gap preferably has a maximal extent of 3 nm or less, preferably 2 nm or less, yet more preferably 1 nm or less. The shorter the gap, the smaller the thickness of the electrolyte liquid present in this gap, and hence the less screening this electrolyte can effectuate between the nucleotide polyphosphate being incorporated in the growing new polynucleotide strand, and the channel of the sensor. To obtain such a small gap, and hence a maximal distance of 3 nm or less, preferably 2 nm or less, yet more preferably 1 nm or less between the part of the polymerase closest to the dielectric region and the dielectric region, a linker and/ or a binding layer as described herein can be used. In embodiments, the part of the polymerase closest to the dielectric region may be within 2 nm, preferably within 1 nm of the dielectric region. This means that in embodiments, the polymerase may be separated from the dielectric region by a binding layer and/or a linker whose combined height is 3 nm or less, 2 nm or less, or 1 nm or less. In embodiments, the part of the enzyme closest to the dielectric region may be in contact therewith. One way to achieve this is to use an enzyme having a hydrophilic part on one hand, and a hydrophilic dielectric region on another hand. A greater proximity between the polymerase and the channel translates into a greater sensitivity.

A second sensitizing means is the use of an electrolyte-screening layer. The electrolyte screening layer is a layer bound to the dielectric region and which fills the gap and/or covers part of the polymerase.

In embodiments, the binding layer (e.g. the self-assembled monolayer or the electrografted layer) may be further bound to molecules forming the electrolyte-screening layer on the binding layer. For instance, the binding layer may form an interface layer between on one hand the dielectric region and on another hand the polymerase and the electrolyte-screening layer.

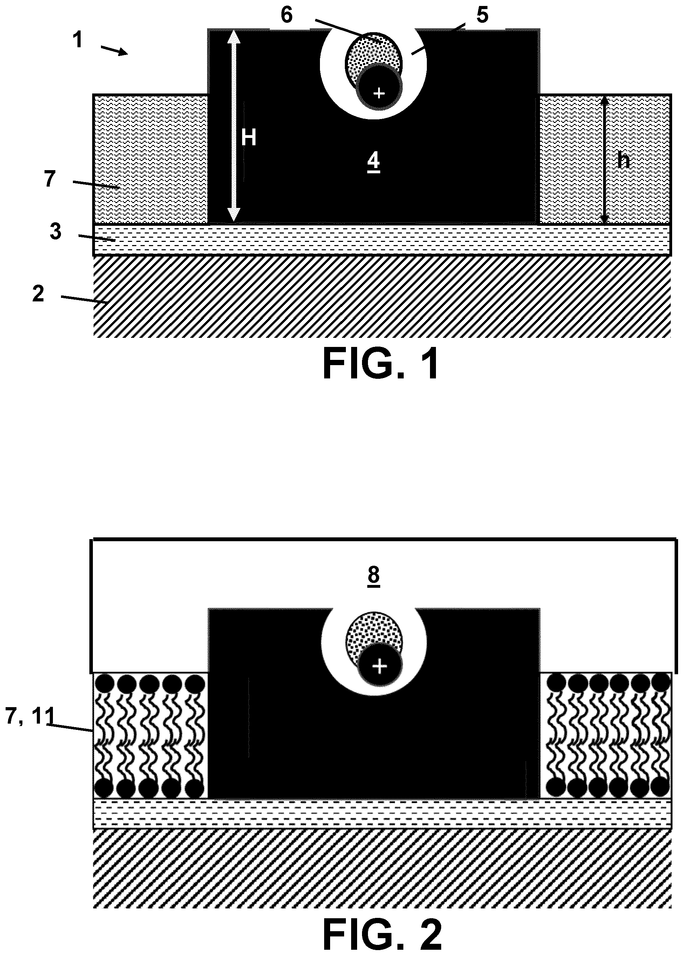

In embodiments, the height of the electrolyte-screening layer may be such that more than 10%, preferably more than 20%, more preferably more than 30%, yet more preferably more than 50% of the height (H, see Fig.1) of the polymerase is covered. In yet more preferred embodiments, the height of the electrolyte-screening layer may be such that more than 75% or more than 90% of the height of the polymerase is covered. This means that if the polymerase is linked to the dielectric region by a linker (or by a binding layer if no linker is present and the binding layer serves as linker, or by a binding layer and a linker if the linker links the polymerase to a binding layer), the height (h, see Fig. 1) of the electrolyte-screening layer may be equal to the height of the linker (or of a binding layer, or of both a linker and a binding layer) plus more than 10, 20, 30, 50, 75 or 90% of the height of the polymerase. Covering a larger height H of the polymerase is advantageous because the higher the electrolyte-screening layer, the less the electrolyte can cause screening of the substrate (target molecule). The active site is left uncovered so that the substrate can still interact with the active site. In embodiments, in order to leave the active site uncovered and in order to avoid preventing the substrates (e.g. target polynucleotide and nucleotide polyphosphate) to reach the polymerase, the height of the electrolyte-screening layer may be chosen to be such that less than 100% of the height of the polymerase is covered.

In embodiments, the electrolyte-screening layer may cover at least part of the polymerase while leaving the active site uncovered, thereby permitting the nucleotide polyphosphate to interact with the active site.

In embodiments, the height of the electrolyte-screening layer may be such that from 2 to 10 nm of the height of the polymerase is covered. Typical heights of oligo ethylene glycol SAMs are 1.2 nm for a trimer, 2.0 nm for a hexamer. These values can help to guide the person skilled in the art in selecting the length of the molecules forming the electrolyte-screening layer (e.g. in function of the size of the used polymerase).

The electrolyte-screening layer is preferably such that the activity of the polymerase is not suppressed. Preferably, the electrolyte-screening layer is such that the activity of the polymerase is not reduced. Depending on the polymerase and on the chemical nature of the electrolyte-screening layer, the height of the electrolyte screening layer can be adapted so that the activity of the polymerase is not suppressed. Similarly, the height of the electrolyte screening layer may be adapted so that the activity of the polymerase is not reduced.

To avoid reduction of the polymerase activity as much as possible, the electrolyte-screening layer is preferably flexible. For this reason, monolayers (e.g. self-assembled monolayers or monolayers attached to a self-assembled monolayer), lipid monolayers, and lipid bilayers are preferred. Cross-linked layers and layers having a Tg higher than 20°C are less preferred due to their higher rigidity.

The electrolyte-screening layer is typically an organic layer.

The electrolyte-screening layer works by keeping the electrolyte as far away from the channel as possible. This can be achieved with hydrophilic, hydrophobic or amphiphilic screening layers. In other words, this can be achieved with screening layers comprising hydrophilic, hydrophobic or amphiphilic molecules. The most important function of the electrolyte-screening layer is to physically occupy the place that would otherwise be occupied by the electrolyte.

Although a hydrophobic character provides a better screening than a hydrophilic character, a hydrophilic character permits a better wetting. A better wetting eases the filling of the fluidic region (which typically comprises a cavity that may measure less 150 nm, for instance less than 100 nm) and permits avoiding the formation of bubbles on the dielectric region. Such bubbles are detrimental to the good functioning of the device. For instance, a hydrophilic electrolyte-screening layer, e.g. comprising a polyalkylene glycol such as an oligo or polyethylene glycol (PEG), despite its hydrophilicity, does play a barrier role against the electrolyte and it has the advantage of permitting a better wetting.

In embodiments, the electrolyte-screening layer may double as an anti-fouling layer, i.e. it may prevent non-specific binding of molecules, other than the target, with the dielectric region or the binding layer. For instance, a hydrophilic electrolyte-screening layer, e.g. comprising a polyalkylene glycol such as an oligo or polyethylene glycol (PEG) can play that anti-fouling role.

Preferred to hydrophilic layer is nevertheless a hydrophobic layer. The hydrophobic layer may, for instance, comprise molecules having a hydrocarbon chain having zero or more hydrogen atoms substituted by halogen atoms. Preferred halogen atoms are fluorine atoms.

Preferably, the electrolyte screening layer is formed of molecules attached to the binding layer. For instance, when the binding layer is a SAM with available azide functional groups, the electrolyte-screening layer may be of the formula DBCO-R", wherein DBCO is dibenzocyclooctyne and where R" is an organic chain having a length suitable for covering at least a part of the height of the polymerase, as described above. Examples of organic chains R" are C₁-C30 organic chains. The organic chain may, for instance, comprise C₁-C₃₀ alkanes, C₁-C₃₀ fluoroalkanes, or oligo(ethylene glycol) chains having from 1 to 12 ethylene glycol repeat units. Examples of such molecule composing a binding layer may have the following formula: 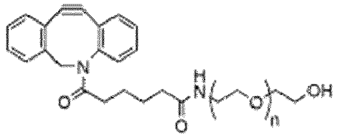 where n is from 1 to 12.

In embodiments, when the electrolyte-screening layer is formed of a self-assembled monolayer of molecules, the density of these molecules on the dielectric region may be from 0.1 to 10 molecules per nm², preferably from 0.4 to 6 molecules per nm², more preferably from 1 to 5 molecules per nm².

Yet more preferred is an amphiphilic layer having a hydrophobic part and a hydrophilic part, with the hydrophilic part being exposed to the fluidic gate region and hence to the electrolyte when the sensor is in operation. For instance, the electrolyte-screening layer may comprise a lipid monolayer or, preferably, a lipid bilayer, such as a cell membrane. In embodiments, a lipid monolayer or bilayer is formed on the dielectric region or on the binding layer. When the electrolyte-screening layer is a lipid monolayer or bilayer, the polymerase is preferably a membrane-associated polymerase.

Methods for forming such a lipid bilayer on a dielectric region such as a SiO₂ region are described in Graneli, Annette, et al. (Langmuir 19.3 (2003): 842-850); C. A. Keller et al. (Phys. Rev. Lett. 84, 5443); and Reimhult et al. (Langmuir 2003 19 (5), 1681-1691). Such methods typically involve the use of small unilamellar proteoliposomes mixed with the protein (in our case a polymerase) of interest and a detergent such as Triton X-100 in order integrate the protein into the proteoliposomes, removing the detergent, then contacting the proteoliposomes containing the protein with the dielectric region or the binding layer.

In embodiments, the electrolyte-screening layer may be bound to the binding layer. In embodiments, both the polymerase and the electrolyte-screening layer may be bound to the binding layer.

The polymerase is typically bound to the binding layer and/or the linker in such a way that the active site is accessible from the aqueous electrolyte, i.e. the active site is exposed to the fluidic gate.

A third sensitizing means that can be used in the present disclosure is the use of an aqueous electrolyte of relatively low conductivity for filing the gap and at least part of the fluidic gate region. The method of the present disclosure operates in an aqueous electrolyte. This electrolyte permits the gate electrode in contact therewith to gate the channel region. For this purpose, this electrolyte must be conductive. The electrolyte also plays a role in untangling the polynucleotide template, thereby preparing it for the sequencing. However, the electrolyte present between the dielectric region and the nucleotide polyphosphate being incorporated into the growing new polynucleotide strand masks the charge of the nucleotide polyphosphate. Due to this screening effect by the electrolyte, the presence of the nucleotide polyphosphate in the active site of the polymerase is difficult to detect. This could maybe be mitigated by the complex method described in WO2014150392 where the intensity of the signal is multiplied by cutting each phosphate bounds in the nucleotide polyphosphate in order to release a corresponding number of protons, that WO2014150392 hopes to detect. To avoid such a complex method, the present inventors realized that one can sensitize the sensor by selecting an electrolyte having a relatively low conductivity. Typically, a conductivity below 0.3 S/m.

The method of the present disclosure can be performed in any electrolyte typically used for polynucleotide sequencing. In particular, salt aqueous solutions can be used. For instance, solutions comprising LiCI, KCI, NaCI, or a mixture thereof can be used. Preferably, the electrolyte is a buffered salt solution. For instance, it may comprise LiCI, KCI, NaCI or a mixture thereof, and additionally comprise a buffer. A typical buffer is a phosphate buffer. A typical example of a suitable electrolyte solution is phosphate-buffered saline (PBS 1 ×) comprising a phosphate buffer concentration of 0.01M and a sodium chloride concentration of 0.154M. The solution pH is 7.4. Such a solution has a conductivity of about 1 S/m.

In embodiments where the electrolyte is used as a sensitizing means, the conductivity of the electrolyte may be adapted to be below 1 S/m, preferably below 0.5 S/m, more preferably below 0.3 S/m. This is advantageous as it reduces the nucleotide polyphosphate screening effect of the electrolyte. In practice, the conductivity of the electrolyte will always be sufficient for the channel to be gated. Indeed, even deionized water left to rest in air has enough CO2 dissolved therein to permit gating. The setting of a lower limit for the conductivity of the electrolyte is therefore not necessary. Hover, as a mere example, the conductivity of the electrolyte may be set above 0.001 S/m. For instance, the conductivity may be above 0.001 but below 1 S/m, above 0.02 but below 0.5 S/m or above 0.03 but below 0.1 S/m. Suitable electrolytes usable as sensitizing means are buffered salt solutions having a conductivity in these ranges. For instance, a phosphate buffer saline with a salt concentration of from 0.00015M to 0.1000M, from 0.0030 to 0.0700M, or from 0.0045 to 0.0150M can be used.

In embodiments, to increase sensitivity, two out of the three sensitizing means may be combined. For instance, a low conductivity aqueous electrolyte as described in any embodiment may be combined with an electrolyte-screening layer as described in any embodiment, or the low conductivity aqueous electrolyte as described in any embodiment may be combined with a short distance between the part of the polymerase closest to the dielectric region and the dielectric region as described in any embodiment (i.e. a short gap), or an electrolyte-screening layer as described in any embodiments can be combined with a short gap as described in any embodiments. In other embodiments, any embodiment of each of all three sensitizing means may be combined.

The fluidic gate region is located above the dielectric region and it is suitable for exposing the active site of the polymerase to the aqueous electrolyte. The fluidic gate region typically is or comprises a cavity in a layer of the sensor, said cavity exposing the active site of the polymerase. In embodiments, the lateral extent of the cavity is typically measuring 150 nm or less, preferably 100 nm or less. The sensor typically has a layer above the dielectric region and this layer has a cavity therein exposing the active site of the polymerase and typically also exposing the entire polymerase and the electrolyte screening layer when present. In embodiments of the first aspect, the fluidic gate region is filled with the aqueous electrolyte. The aqueous electrolyte is typically an electrolyte containing the analyte and serving as a liquid gate. In some embodiments of the second aspect (see below), the fluidic gate region is at least partially empty (of liquid) or not filled with a liquid. In some embodiments of the second aspect, at least part of the fluidic gate region is filled with air. This would, for instance, be the case when the sensor is not in use and no liquid is in the fluidic gate region. In some embodiments, an inlet and an outlet for the liquid are provided to the fluidic gate region. For instance, the inlet may be located on one side of the fluidic gate region and the outlet may be located on the opposing side of the fluidic gate region. Typically, the fluidic gate region is defined at least partially by the dielectric region or any layer thereon, and sidewalls. In some embodiments, a cover is located above the fluidic gate region and the fluidic gate region is defined by the cover, the dielectric region or any layer thereon, and sidewalls (e.g. connecting the cover and the dielectric region or any layer thereon). When a cover is present, an inlet and an outlet can be present in the cover.

The polymerase activity may be monitored electronically which allows sequencing the template polynucleotide.

The sensor of the present disclosure is used for polynucleotide (e.g. DNA or RNA) sequencing. For this purpose, the enzyme may be a polynucleotide polymerase, such as a DNA polymerase. The building in of the nucleotides by the polymerase is electronically monitored which allows determining the sequence of the DNA running through the polymerase. For instance, the change of the nucleotides or the configuration change of the polymerase can be detected.

The sensor typically comprises a solid gate electrode placed in the fluidic gate region in such a way that it is in electrical contact with the liquid electrolyte gate when this one is present. The sensor typically further comprises a source electrode and a drain electrode in electrical contact with respectively the source region and the drain region of the active region of the sensor.

Step b of the method of the present disclosure comprises exposing the polymerase to one or more nucleotide polyphosphates.

Step c of the method of the present disclosure comprises electrically monitoring changes in the channel electrical properties.

In embodiments of the first aspect, the one or more nucleotide polyphosphates do not comprise a terminal blocking group. This is one of the advantages of the method of the present disclosure. Because of the use of one or more sensitizing means, phosphatase is not necessary and hence terminal blocking groups preventing the phosphatase from cleaving phosphate bounds prior to incorporation into the newly growing polynucleotide strand is also not necessary.

In embodiments, the one or more nucleotide polyphosphates may have the general formula: 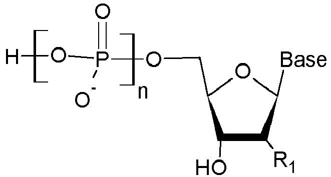 , or an ionic form thereof,
wherein n is at least 3, R₁ is either H or OH and the base is selected from adenine, guanine, cytosine, thymine, and uracil.

In embodiments, the one or more nucleotide polyphosphates may comprise at least a nucleotide polyphosphate comprising a polyphosphate chain of four or more phosphates. In other words, n may be equal to 4 or more in the formula above for at least one of the one or more polyphosphates. In embodiments, each of the one or more nucleotide polyphosphates may independently comprise a polyphosphate chain of four or more phosphates. In other words, n may be independently selected from values equal to 4 or more in the formula above for each of the one or more nucleotide polyphosphates.

In exemplary embodiments, nucleotide polyphosphates of use in methods of the disclosure have at least 4, 5, 6, 7, 8, 9, 10, 11, 12, 13, 14, 15, 16, 17, 18, 19, 20, 21, 22, 23, 24, 25, 26, 27, 28, 29, or 30 phosphate groups. For instance, nucleotide polyphosphates of use in methods of the first aspect may have 4, 5, 6, 7, 8, 9, 10, 11, 12, 13, 14, 15, 16, 17, 18, 19, 20, 21, 22, 23, 24, 25, 26, 27, 28, 29, 30 phosphate groups. Preferably, the methods of the disclosure described herein do not utilize nucleotide triphosphates (i.e. nucleoside polyphosphates with three phosphate groups). It is advantageous to use nucleotide polyphosphates having at least 4 phosphate groups because they comprise at least four charges which make them easier to detect by the sensor.

In embodiments, the one or more nucleotide polyphosphates may comprise at least a nucleotide polyphosphate comprising a polyphosphate chain of from 3 to 30 phosphates, preferably from 4 to 30 phosphates. In other words, n may be from 3 to 30, preferably from 4 to 30 for at least one of the one or more nucleotide polyphosphate. In embodiments, each of the one or more nucleotide polyphosphates may independently comprise a polyphosphate chain of from 3 to 30 phosphates, preferably from 4 to 30 phosphates. In other words, n may be independently selected from values ranging from 3 to 30, preferably from 4 to 30 in the formula above for each of the one or more nucleotide polyphosphates.

One advantage of the methods of the first aspect is that the use of nucleotide polyphosphates having a terminal protecting group is not necessary. However, they may be used and may be as described in WO2014150392

Another advantage of embodiments of the present disclosure is that the nucleotide polyphosphates may be label-free. In other words, they can be free of any group, other than the phosphate groups, aiming at detecting the nucleotide. For instance, they may be free of a dye, of a radioactive marker, of a fluorophore, or of a magnetic tag. However, such a label can be used as described in WO2014150392 [0058].

As will be discussed in further detail herein, methods of the disclosure utilize one or more types of nucleotide polyphosphates. In some embodiments, each of the different types of nucleotides will have a different number of phosphate groups in the polyphosphate chain, such that each type may be identified from each other type upon incorporation. For example, each of the different types of nucleotide polyphosphates may each correspond to a nucleobase independently selected from A, G, C, T or U (or to one or more modified nucleobases), and each type may be distinguished from the other types based on characteristics such as the signal generated when the nucleotide analog is incorporated during a polymerase reaction. For example, each type of nucleotide analog can in some embodiments have a different number of phosphate groups in the polyphosphate chain, such that, upon incorporation of a particular nucleotide analog type during a polymerization reaction, the signal associated with the presence of the n charges on the polyphosphate group of the nucleotide in close proximity with the channel will identify the incorporated nucleotide analog as having a nucleobase A, C, G, T, or U (A, C, G, T in the case of DNA and A, C, G, or U in the case of RNA). In further embodiments, sequencing reactions discussed herein may utilize one or more different types of nucleotide polyphosphates, and in further exemplary embodiments, each of the different types of nucleotide polyphosphate has a different number of phosphate groups in their polyphosphate chains.

In addition to the naturally occurring "nucleobases," adenine, cytosine, guanine, and thymine (A, C, G, T, U), nucleic acid components of the nucleotide polyphosphate of the first aspect optionally include modified bases. These components can also include modified sugars. For example, the nucleic acid can comprise at least one modified base moiety which is selected from the group including, but not limited to, 5-bromouracil, 5-fluorouracil, 5- chlorouracil, 5-iodouracil, xanthine, hypoxanthine, 4-acetylcytosine, 5-carboxymethylaminomethyl-2-thiouridine, 5-(carboxyhydroxylmethyl) uracil, 5-carboxymethylaminomethyluracil, beta-D-galactosylqueosine, dihydrouracil, inosine, N<6>- isopentenyladenine, 1-methylguanine, 2,2-dimethylguanine, 1-methylinosine, 2- methyladenine, 2-methylguanine, 3-methylcytosine, 5-methylcytosine, N<6>-adenine, 5-methylaminomethyluracil, 5-methoxyaminomethyl-2-thiouracil, 7-methylguanine, beta-D-mannosylqueosine, 5'-methoxycarboxymethyl uracil, 2-methylthio-N<6>-isopentenyladenine, 5-methoxyuracil, uracil-5-oxyacetic acid (v), pseudouracil, wybutoxosine, queosine, 5-methyl-2-thiouracil, 2-thiocytosine, 2-thiouracil, 5-methyluracil, uracil-5-oxyacetic acid methyl ester, uracil-5-oxyacetic acid (v), 5-methyl-2-thiouracil, 4-thiouracil, 3-(3-amino-3-N-2-carboxypropyl) uracil, (acp3)w, nitroindole, and 2,6-diaminopurine.

In further embodiments, nucleotide polyphosphates of the present disclosure may include nucleotide polyphosphates having the structure:

P-S-B,

wherein B is a natural or non-natural nucleobase, S is selected from a sugar moiety, an acyclic moiety or a carbocyclic moiety, and P is a modified or unmodified polyphosphate.

The base moiety, B, incorporated into the nucleotide polyphosphates of the present disclosure is generally selected from any of the natural or non-natural nucleobases or nucleobase analogs, including, e.g., purine or pyrimidine bases, such as those that are routinely found in nucleic acids and nucleic acid analogs, including adenine, thymine, guanine, cytidine, uracil, and in some cases, inosine. For purposes of the present description, nucleotides and nucleotide analogs are generally referred to based upon their relative analogy to naturally occurring nucleotides. As such, an analog that operates, functionally, like adenosine triphosphate, may be generally referred to herein by the shorthand letter A. Likewise, the standard abbreviations of T, G, C, U and I, may be used in referring to analogs of naturally occurring nucleosides and nucleotides typically abbreviated in the same fashion. In some cases, a base may function in a more universal fashion, e.g., functioning like any of the purine bases in being able to hybridize with any pyrimidine base, or vice versa. The base moieties used in the present disclosure may include the conventional bases described herein or they may include such bases substituted at one or more side groups, or other fluorescent bases or base analogs, such as 1 ,N6 ethenoadenosine or pyrrolo C, in which an additional ring structure renders the B group neither a purine nor a pyrimidine. For example, in certain cases, it may be desirable to substitute one or more side groups of the base moiety with a labeling group or a component of a labeling group, such as one of a donor or acceptor fluorophore, or another labeling group. Examples of labeled nucleobases and processes for labeling such groups are described in, e.g., U.S. Pat. Nos. 5,328,824 and 5,476,928, each of which is incorporated herein by reference in its entirety for all purposes and in particular for all teachings related to nucleobases and labeling nucleobases.

In the nucleotide polyphosphates of the present disclosure, the S group is generally a sugar moiety that provides a suitable backbone for a synthesizing nucleic acid strand. In it most preferred aspect, the sugar moiety is selected from a D-ribosyl, 2' or 3' D-deoxyribosyl, 2',3'-D-dideoxyribosyl, 2',3'-D-didehydrodideoxyribosyl, 2' or 3' aminoribosyl, 2' or 3' alkoxyribosyl, 2' or 3' mercaptoribosyl, 2' or 3' alkothioribosyl, acyclic, carbocyclic or other modified sugar moieties. A variety of acyclic or carbocyclic moieties may be incorporated as the "S" group in place of a sugar moiety, including, e.g., those described in published U.S. Patent Application No. 2003/0124576, incorporated herein by reference in its entirety for all purposes and in particular for all teachings related to sugar moieties of nucleotides and nucleotide analogs.

The P groups in the nucleotides of the disclosure are modified or unmodified polyphosphate groups. As discussed above, the number of phosphates in the polyphosphate can be 4, 5, 6, 7, 8, 9, 10, 1 1 , 12, 13, 14, 15, 16, 17, 18, 19, 20, 21 , 22, 23, 24, 25, 26, 27, 28, 29, or 30 phosphate groups or modified phosphate groups. The unmodified phosphates have linearly linked - O-P(O)₂- units, for example a tetraphosphate, pentaphosphate, hexaphosphate, heptaphosphate, or octaphosphate. The P groups also include modified polyphosphates as describe in WO2014/150392 [0062-0064].

In embodiments, the B, S, and P groups can be connected directly, or can be connected using a linking unit such as an -O-, -S-, -NH-, or -CH2- unit.

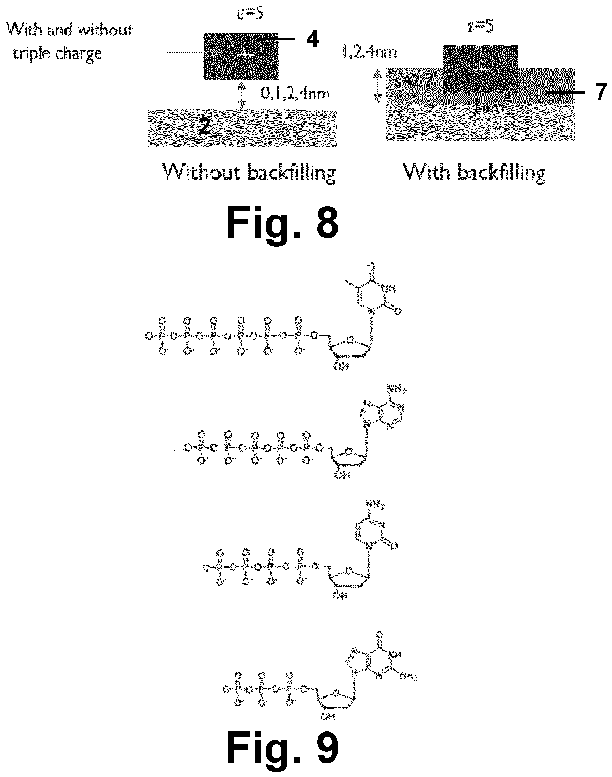

In embodiments, each nucleotide polyphosphate used in the method and differing by its base, also differs by its number n of phosphate groups. This is advantageous because each different nucleotide carries a different charge, which can be discriminated by the sensor. The sensor is accordingly able to read which type of nucleotide is being built into the DNA strand by recognizing the charge of the nucleotide. As a result, no labels need to be attached to the nucleotide polyphosphate, it is the nucleotide polyphosphate itself that carries the message of its identity. Since nucleotides already incorporated had their polyphosphate group removed upon incorporation, only the nucleotide polyphosphate currently being incorporated has its n-times charged polyphosphate group present, permitting its identification by the sensor. Fig. 9 shows examples of nucleotide polyphosphate for use with the present disclosure. This figure only represents an example and the nature of the base associated with a particular polyphosphate length can of course, for instance, be exchanged with a different base currently associated with a different particular polyphosphate length.

In embodiments, step b) may comprise exposing the polymerase to a single type of nucleotide polyphosphate of a first base while step c of electrically monitoring changes in the channel electrical properties is performed. The method may repeat steps b) and step c), by substituting the single type of nucleotide polyphosphate of a first base by a single type of nucleotide polyphosphate of a different base than the base in the just used nucleotide polyphosphate for each new repeat of steps b) and c). In these embodiments, each nucleotide polyphosphate may have a number of phosphate group of 4 or more, preferably 5 or more, yet more preferably 6 or more. A larger number of phosphate group increases sensitivity. All of the nucleotide polyphosphates would have such a large charge then. Only one nucleotide is present in the different repeats, e.g. first A, then T, then G, then C. In each repeat it is detected whether one or more nucleotide of a particular base is built in. This embodiment may comprise a step of recording which nucleotide polyphosphate is used for each repeat.

In alternative embodiments of the first aspect, step b) may comprise simultaneously exposing the polymerase to a first type of nucleotide polyphosphate of a first base, and to a second type of nucleotide polyphosphate of a second base, different from the first base, the first type of nucleotide polyphosphate comprising a polyphosphate chain of at least one more phosphates, preferably at least two more phosphates, yet more preferably at least 3 more phosphates than a polyphosphate chain of the second type of nucleotide polyphosphate, and wherein the method repeats steps b) and c), by substituting the nucleotide polyphosphates just used by nucleotide polyphosphates of other bases, said bases being different from each other, for each new repeat. For instance, a nucleotide triphosphate and a nucleotide hexaphosphate, further differing in the nature of their base, could be used simultaneously. Only 2 of the 4 nucleotides are present simultaneously in a repeat. For instance, in a first repeat A and G could be used, and then in the subsequent repeat, only T and C would be used. This would lead to e.g. a sequence of (A, G) elements in the first repeat. For instance, both A and T could have 3 phosphates and T and C could have 6 phosphates in their respective polyphosphate groups. These alternative embodiments, however, require that the sensor be able to distinguish charge differences between the different nucleotide polyphosphates. This is made possible by the present disclosure as it makes use of one or more sensitizing means. In embodiments, to increase sensitivity, two out of the three sensitizing means, or even all three of them, may be combined as explained above. A further strategy to increase sensitivity is to use simultaneously four nucleotide polyphosphates differing from each other in the length of their polyphosphate group by at least two phosphate groups or even more preferably by at least three phosphate groups. This alternative embodiment may comprise a step of recording which pair of nucleotide polyphosphate is used for each repeat.

In further alternative embodiments of the first aspect, step b) may comprise simultaneously exposing the polymerase to four types of nucleotide polyphosphate, each type having a different base and a different number of phosphates in a polyphosphate chain thereof. In this single pot version, the solution does not need to be cycled and sequencing can be done continuously. These further alternative embodiments, however, require that the sensor be able to distinguish charge differences between the different nucleotide polyphosphates. This is made possible by the present disclosure as it makes use of one or more sensitizing means. In embodiments, to increase sensitivity, two out of the three sensitizing means, or even all three of them, may be combined as explained above. A further strategy to increase sensitivity is to use simultaneously four nucleotide polyphosphates differing from each other in the length of their polyphosphate group by at least two phosphate groups or even more preferably by at least three phosphate groups. An advantage of this further alternative embodiment of the first aspect is that there is no need to record which nucleotide polyphosphate is used since there is no cycle.

In a second aspect, the present disclosure relates to a sensor according to any embodiments of the first aspect.

Any feature of the second aspect can be as correspondingly described in the first aspect.

In a third aspect, the present disclosure relates to a method for manufacturing a sensor. The method comprises forming a field effect transistor (FET). Forming the FET comprises:
- providing an active region comprising a source region, a drain region, and a channel region between the source region and the drain region,
- providing a dielectric region on the channel region,
- coupling a polymerase to the dielectric region, the polymerase having an active site for interacting with a substrate,
- coupling an electrolyte-screening layer to the dielectric region so that the electrolyte-screening layer covers at least part of the polymerase while leaving the active site uncovered, thereby permitting interaction of the substrate with the active site, and
- forming a fluidic gate region to which the active site of the polymerase is exposed.

In embodiments, forming the FET comprises:
- providing an active region comprising a source region, a drain region, and a channel region between the source region and the drain region,
- providing a dielectric region on the channel region,
- forming a fluidic gate region exposing the dielectric region,
- coupling a polymerase to the dielectric region and in the fluidic gate region, the polymerase having an active site for interacting with a substrate, the active site of the polymerase being exposed to the fluidic gate region,
- coupling an electrolyte-screening layer to the dielectric region and in the fluidic gate region, so that the electrolyte-screening layer covers at least part of the polymerase while leaving the active site uncovered, thereby permitting interaction of the substrate with the active site.

In any embodiments of the third aspect, forming a fluidic gate region may comprise:
- providing a sacrificial gate electrode on the dielectric region,
- providing an overlayer on the sacrificial gate electrode, the source region, and the drain region,
- forming a cavity in the overlayer, thereby exposing the sacrificial gate electrode, and
- removing the sacrificial gate electrode, thereby forming the fluidic gate region.

The sacrificial gate electrode is formed of a material that can be etched selectively with respect to the overlayer and the dielectric region. For instance, the sacrificial gate electrode may be made of poly-Si.

The overlayer may, for instance, be an oxide layer such as a SiO₂ layer.

Forming the cavity in the overlayer can, for instance, be performed by an etching process suitable to selectively etch the overlayer with respect to the sacrificial gate electrode. For instance, a plasma etching process can be used.

Removing the sacrificial gate electrode may comprise a wet etch step. For instance, the sacrificial gate electrode may be physically contacted with an etching liquid suitable for selectively etching the sacrificial gate electrode with respect to the overlayer and the dielectric region. In the case of a poly-Si sacrificial gate and of an oxide overlayer and an oxide dielectric region, a tetramethylammonium hydroxide (TMAH) solution can be used to remove the sacrificial gate.

In embodiments, coupling the polymerase and the electrolyte-screening layer to the dielectric region may comprise a preliminary step of forming a binding layer on the dielectric region. The binding layer may be as defined in the first aspect. For instance, the binding layer may suitable for, on one hand, binding to the dielectric region, and on another hand, binding to the polymerase.

A typical example of binding layer may be a self-assembled monolayer of molecules comprising a trimethoxysilane group for binding with the dielectric region and an azide group for binding with the polymerase. Such a binding layer may, for instance, be deposited in an oven from the vapor phase.

Once the binding layer is formed, it is preferable to first bind the polymerase therewith and then binding the electrolyte-screening layer therewith.

Binding the polymerase with the binding layer is preferably performed in such a way that only one polymerase is present in the fluidic gate region on the binding layer. To achieve this, Poisson loading can be used as is well known to the person skilled in the art. For instance, Poisson loading may comprise exposing the binding layer with a volume of liquid having from 10 to 50% (typically from 20 to 37%) of chances of comprising a single polymerase. This typically results in either one or zero polymerase bound to the binding layer. In the case of a biosensing device comprising a plurality of sensors according to the first aspect, Poisson loading may comprise exposing the plurality of sensors to a volume of liquid having a polymerase concentration such that 10 to 50% (typically from 20 to 37%) of the sensors comprise a single polymerase in their fluidic gate region.

The binding of the polymerase on the binding layer is preferably selective. Selectivity can, for instance, be achieved by using a polymerase having a linker attached therewith, said linker having an A group (such as an alkyne group) as defined in the first aspect. When the polymerase comprises a linker comprising an alkyne group or a strained alkyne group, that group can, for instance, be reacted with azide groups of the binding layer. The reaction can be performed in the conditions of click chemistry, e.g. in the presence (for an unstrained alkyne) or absence (for a strained alkyne) of a Cu catalyst.

Binding the electrolyte-screening layer to the binding layer can, for instance, be achieved by contacting the binding layer with a liquid comprising polyethylene glycol molecules comprising an alkyne or a strained alkyne such as a dibenzocyclo-octyne group. The reaction can be performed in the conditions of click chemistry, e.g. in the presence (for an unstrained alkyne) or absence (for a strained alkyne) of a Cu catalyst.

Any feature of the third aspect can be as correspondingly described in the first or second aspect.

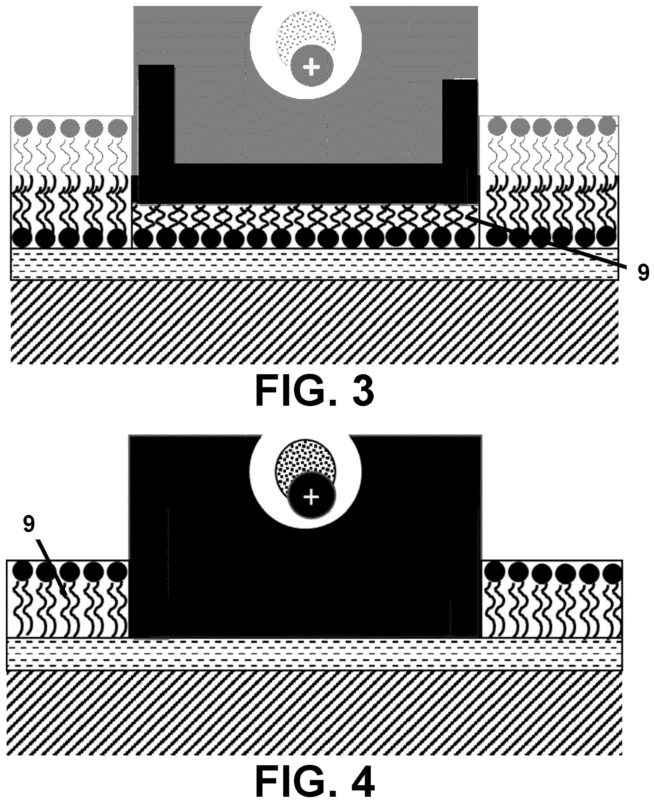

Figs. 1 to 4 are schematic illustrations of vertical cross-sections of embodiments of the present disclosure. In Fig.1, a sensor (1) is depicted having a polymerase (4) of height (H) mounted on a binding layer (3), itself bounded to the dielectric region (2) of the FET. An electrolyte-screening layer (7) of height (h) is wrapping the mounted polymerase and covers part thereof. An active site (5) is depicted and a substrate (6) having a positive charge is shown inside the active site. Fig. 2 is analogous to Fig. 1 but the electrolyte-screening layer (7) is a lipid bilayer (11). Fig. 2 also depicts the fluidic gate region (8). Fig. 3 is analogous to Fig. 2 but a lipid monolayer (9) is used between the binding layer and the polymerase to provide a good barrier against the electrolyte. Fig. 4 is analogous to Fig. 2 but the lipid bilayer is replaced by a lipid monolayer (9).

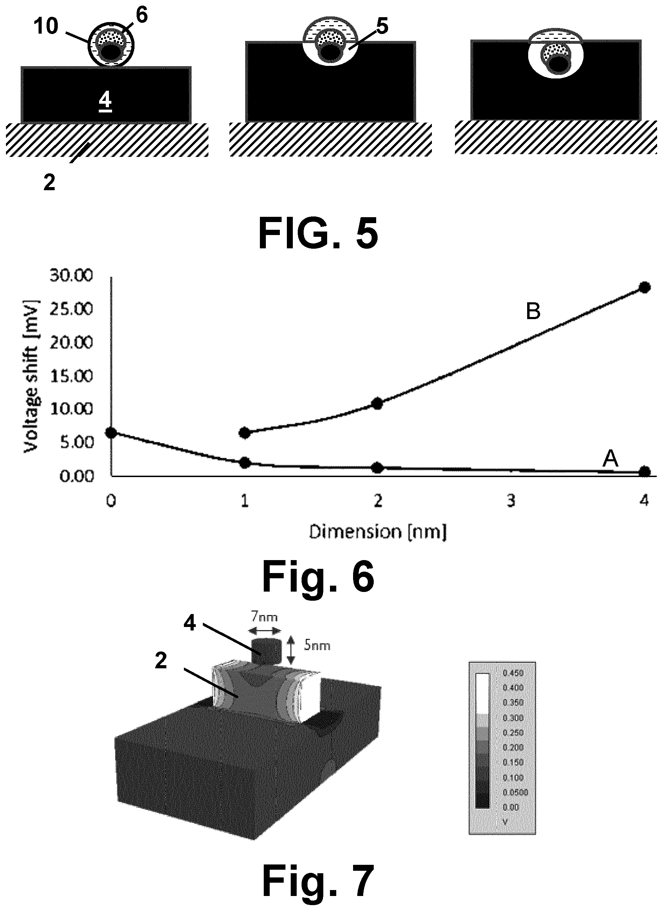

Fig. 5 is a schematic illustration of vertical cross-sections of embodiments of the present disclosure. On the left, in a less preferred embodiment, a bonded substrate (6) is shown which is not enveloped by the polymerase (4) and hence strongly screened from the channel by the surrounding electrolyte (10). In the two illustrations further to the right, the polymerase is selected in such a way that the active site (5) envelops the substrate to some degree and thereby expel the electrolyte present between the substrate and the polymerase body. The choice of the polymerase and its orientation on the FET dielectric region (2) are such that screening by the electrolyte (10) is prevented to a significant extent. Different depths of enveloping binding pockets are illustrated. The envelopment allows reducing screening by the electrolyte.

Fig. 6 shows a graph of the voltage shift (mV) observed when a polymerase captures a charged substrate during A) Technology Computer Aided Design (TCAD) simulations of comparative examples and B) TCAD simulations of embodiments of the present disclosure. The voltage shift is due to three elementary charges. The simulated channel length, width, and height were respectively 25 nm, 10 nm, and 10 nm. The presence of a 10x diluted PBS electrolyte was simulated as well.

Fig. 7 is a three-dimensional illustration of the simulated sensor showing the silicon body of the channel (2) and the polymerase (4) modeled as a cylinder. Backfilling is not shown. The calculated voltage is shown in grayscale.

Fig. 8 is a schematic illustration of the parameters used for the TCAD simulation with and without backfilling.

For the comparative examples, a polymerase was placed in an electrolyte at a distance of 0, 1, 2, 3, or 4 nm of the dielectric region of the sensor. As can be seen in curve A of Fig. 6, the voltage shift quickly decreases with the distance to the dielectric region, in large part due to the presence of electrolyte between the polymerase and the dielectric region.

For the examples according to the present disclosure, a polymerase was kept at a constant distance of 1 nm with respect to the dielectric region but an electrolyte-screening layer of thickness 1, 2 or 4 nm was used. When the thickness was 1 nm, the space between the polymerase and the dielectric region was filled with the electrolyte-screening layer but the height of the polymerase was not even partially covered. When the thickness was 2 or 4 nm, the height of the polymerase was covered at respectively 20% and 60% respectively. As can be seen in Curve B of Fig. 6, the higher the polymerase height covered, the higher the voltage shift.

### Example: Electrical sequencing of a DNA template

### 0) Preparation of a DNA sequencing library

This step is a first step in the formation of a DNA template. It is performed as is well known to the person skilled in the art. It comprises a step of purification of a DNA sample, random fragmentation of the DNA, DNA repair, and 5' and/or 3' adapter ligation of the DNA. Alternatively, "tagmentation" can be used, thereby combining the fragmentation and ligation reactions into a single step. Adapter-ligated fragments are then amplified by PCR and then purified by gel or capillary electrophoresis. These steps being part of the state of the art, they are not detailed here.

### 1) Preparation of the sensor

### a. First option: loading of the polymerase-DNA complexes on the dielectric

In this embodiment, a purified DNA template obtained in step 0 is first bound to the polymerase and this complex is bound to the dielectric region.

### a1. Preparation of a DNA-polymerase binding mix

(i) In this step, the buffer containing the DNA template from the DNA library is changed for a Tris buffer,
(ii) The DNA template and a primer are annealed together as is well known in the art,
(iii) A Φ29 DNA polymerase is modified by reacting it with 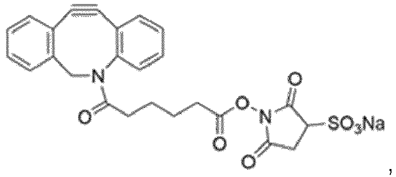 then by purifying it,
(iv) The modified Φ29 DNA polymerase is then put in presence of the DNA template-primer assembly, thereby binding the polymerase to said assembly as is well known in the art,
(v) The obtained DNA-polymerase complex is then purified and its concentration in the Tris buffer is adapted to allow for Poisson loading as is well known in the art and as will be briefly explained next.

### a2. Poisson loading of the DNA-polymerase complex onto the dielectric region

(i) The DNA-polymerase in its Tris buffer is contacted with a plurality of sensors, each having its own dielectric region. Each dielectric region has a binding layer thereon, said binding layer having azide functional groups for reacting with the DBCO group of the modified Φ29 DNA polymerase. As a result of (preferably optimal) Poisson loading, about one dielectric region on three has a single DNA-polymerase complex bound to it.
(ii) The fluidic gate cavity of the sensor is rinsed with a buffer.

### a3. Sequencing

At this point, the solution in the fluidic gate cavity is changed to one that is appropriate for sequencing. For instance, an electrolyte aqueous solution (e.g. PBS 0.5x) may be used. The sensor is turned on and from this moment the sensor is actively monitoring the polymerase enzymes as the solution is changed and the sequencing starts. The solution in the fluidic gate which will allow for the sequencing to commence may, for instance, contain MgCl₂ (a polymerase cofactor) and the modified polyphosphate nucleotides displayed in Figure 9.

### b. Second option: loading of the polymerase alone on the dielectric region, followed by forming the polymerase - DNA complex

In this embodiment, a polymerase is first bound to the dielectric region, and only later is the polymerase annealed with a purified DNA template.

### b1. Prepare polymerase binding mix

(i) In this step, the buffer containing the Φ29 DNA polymerase is changed for a Tris buffer,
(ii) The polymerase is modified by reacting it with 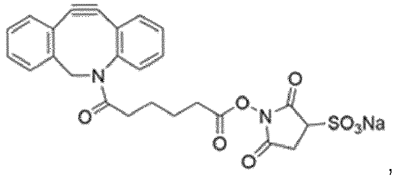 then by purifying it,
(iii) the concentration of the polymerase in the Tris buffer is adapted to allow for Poisson loading at the optimal concentration for Poisson loading as is well known in the art and as will be briefly explained next,

### b2. Poisson loading of polymerase onto the dielectric region

(i) The polymerase in its Tris buffer is contacted with a plurality of sensors, each having its own dielectric region. Each dielectric region has a binding layer thereon, said binding layer having azide functional groups for reacting with the DBCO group of the modified Φ29 DNA polymerase. As a result, about one dielectric region on three have a single modified polymerase bound to it,
(ii) The fluidic gate cavity of the sensor is rinsed with a buffer,

### a3. sequencing

At this point, the solution in the fluidic gate cavity is changed to one that is appropriate for sequencing. For instance, an electrolyte aqueous solution (e.g. PBS 0.5x) may be used. The sensor is turned on and from this moment the sensor is actively monitoring the polymerase enzymes as the solution is changed and the sequencing starts. The solution in the fluidic gate which will allow for the sequencing to commence may, for instance, contain the DNA template, MgCl₂ (a polymerase cofactor), and the modified polyphosphate nucleotides displayed in Figure 9. First, the DNA template will hybridize with the polymerase, then the sequencing starts.

It is to be understood that although preferred embodiments, specific constructions, and configurations, as well as materials, have been discussed herein for devices according to the present disclosure, various changes or modifications in form and detail may be made without departing from the scope and spirit of this disclosure. For example, any formulas given above are merely representative of procedures that may be used. Functionality may be added or deleted from the block diagrams and operations may be interchanged among functional blocks. Steps may be added or deleted to methods described within the scope of the present disclosure.

## Claims

1. A method for sequencing a template polynucleotide, comprising the steps of:
a) Providing a sensor (1) comprising:
- An active region comprising a source region, a drain region, and a channel region (2) between the source region and the drain region,
- A dielectric region (3) on the channel region (2),
- A polymerase (4) coupled to the dielectric region (3), the polymerase having an active site (5) for template polynucleotide entry, nucleotide polyphosphate entry, and newly synthesized polynucleotide output, the polymerase (4) being separated from the dielectric region (3) by a gap,
- One or more sensitizing means selected from;
∘ an aqueous electrolyte filling said gap and at least part of the fluidic gate region (8), the aqueous electrolyte having a conductivity below 0.3 S/m,
∘ an electrolyte-screening layer (7) bound to the dielectric region (3), and
∘ a maximal distance of 3 nm or less, preferably 2 nm or less, between the part of the polymerase (4) closest to the dielectric region (3) and the dielectric region (3),
- A fluidic gate region (8) to which the polymerase (4) is exposed,
- A template polynucleotide bound to a primer, the template polynucleotide being bound to the polymerase (4),
b) Exposing the polymerase (4) to one or more nucleotide polyphosphates; and
c) Electrically monitoring changes in the channel electrical properties.

2. The method according to claim 1, wherein the low conductivity medium is a buffered salt solution.

3. The method according to claim 1 or claim 2, wherein the electrolyte-screening layer (7) covers part of the polymerase (4) while leaving the active site (5) uncovered.

4. The method according to any one of the preceding claims, wherein the height of the electrolyte-screening layer (7) is such that more than half of the height of the polymerase (4) is covered.

5. The method according to any one of the preceding claims, wherein the electrolyte-screening layer (7) comprises a lipid bilayer (11).

6. The method according to claim 5, wherein the lipid bilayer (11) is a cell membrane.

7. The method according to any of the preceding claims, wherein the height (h) of the electrolyte-screening layer (7) is such that from 2 to 10 nm of the height (H) of the polymerase (4) is covered.

8. The method according to any one of the preceding claims, wherein the maximal distance between the part of the polymerase (4) closest to the dielectric region (3) and the dielectric region (3) is 1 nm or less.

9. The method according to any one of the preceding claims, wherein the one or more nucleotide polyphosphates do not comprise a terminal blocking group.

10. The method according to any one of the preceding claims, wherein the one or more nucleotide polyphosphates have the general formula: 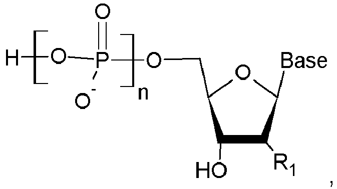 or an ionic form thereof,
wherein n is at least 3, R₁ is either H or OH and the base is selected from adenine, guanine, cytosine, thymine, and uracil.

11. The method according to any one of the preceding claims, wherein the one or more nucleotide polyphosphates comprise at least a nucleotide polyphosphate comprising a polyphosphate chain of four or more phosphates.

12. The method according to any one of the preceding claims, wherein step b) comprises exposing the polymerase to a single type of nucleotide polyphosphate of a first base, and wherein the method repeats steps b) and c), by substituting the single type of nucleotide polyphosphate of a first base by a single type of nucleotide polyphosphate of a different base than the base in the just used nucleotide polyphosphate for each new repeat.

13. The method according to any one of claims 1 to 11, wherein step b) comprises simultaneously exposing the polymerase to a first type of nucleotide polyphosphate of a first base, and to a second type of nucleotide polyphosphate of a second base, different from the first base, the first type of nucleotide polyphosphate comprising a polyphosphate chain of at least one more phosphates, preferably at least two more phosphates than a polyphosphate chain of the second type of nucleotide polyphosphate, and wherein the method repeats steps b) and c), by substituting the nucleotide polyphosphates just used by nucleotide polyphosphates of other bases, said bases being different from each other, for each new repeat.

14. The method according to any one of claims 1 to 11, wherein step b) comprises simultaneously exposing the polymerase to four types of nucleotide polyphosphate, each type having a different base and a different number of phosphates in a polyphosphate chain thereof.

15. The method according to any one of the preceding claims, wherein the channel region has a length of 10 to 150 nm and/or a width of 3 to 20 nm and/or a height of 5 to 40 nm.
